# EUROPEAN PATENT SPECIFICATION

(11) **EP 1 643 834 B1**
(45) Date of publication and mention of the grant of the patent: **01.08.2007**
(21) Application number: 04736668.7
(22) Date of filing: 11.06.2004
(51) Int. Cl.: A01N 25/30, A01N 43/40, A01N 57/20, C07C 217/02

(54) **SURFACTANT COMPOUNDS AND AGROCHEMICAL COMPOSITIONS**
TENSIDVERBINDUNGEN UND AGROCHEMISCHE ZUSAMMENSETZUNGEN
COMPOSES TENSIOACTIFS ET COMPOSITIONS AGROCHIMIQUES

(30) Priority: 16.06.2003 GB 0313829
(43) Date of publication of application: 12.04.2006
(73) Proprietor: CRODA INTERNATIONAL PLC, Goole East Yorkshire DN14 9AA (GB)
(72) Inventor: BEVINAKATTI, Hanamanthsa Shankarsa, Yarm, Cleveland TS15 9EW (GB)
(74) Representative: Roberts, Jonathan Winstanley
(86) International application number: PCT/GB2004/002525
(87) International publication number: WO 2004/112478

(56) References cited:
- WO-A-01/05224
- DE-A- 4 238 214

## Description

This invention relates to surfactant compounds which are alkoxylated derivatives of compounds including a polyhydroxy hydrocarbyl, particularly a saccharide, amine residue, a hydrophobic residue and a linking group, particularly including a glycidyl group, and to the use of such compounds as surfactants in agrochemicals.

Surfactants are widely used in agrochemical compositions and formulations as adjuvants. Adjuvants act to increase the effect of agrochemicals (by a variety of possible mechanisms).

Surfactants including polyhydroxy hydrocarbyl, particularly saccharide, substituents, particularly as amides have been suggested e.g. for cleaning applications. Other surfactant compounds including polyhydroxy hydrocarbyl and amino groups are disclosed in JP 54163829 A to fatty alcohol glycidyl amine glucoside derivatives in making cosmetic emulsions; DE 4238214 A and DE 4238215 A to fatty glycidyl amine glucoside derivatives in making polyurethane materials; DE 4238216 A and

DE 4238217 A to quaternary derivatives of such materials as textile surfactants and DE 4307475 A to betaine derivatives. Our PCT Application WO 01/05224 A describes polyhydroxycarbyl fatty glycidyl amine derivatives, particularly fatty ethoxylate glycidyl amine glucosides, and their use especially as adjuvants in agrochemical formulations. These materials can be effective adjuvants, but the better materials require intermediates based on glycidyl ethers of fatty alcohol ethoxylates which are not readily available commercially and so the final products are relatively expensive.

This invention is based on the finding that certain surfactants which are alkoxylated derivatives of compounds including polyhydroxy hydrocarbyl, particularly saccharide, amine groups can be useful in agrochemical applications, compositions and formulations, in particular providing adjuvancy.

The compounds of and used in this invention can:
1 provide enhanced activity for agrochemicals, especially water soluble herbicides, notably in terms of enhancing the speed of effectiveness; and
2 have significantly lower aquatic toxicity than conventional surfactants used in agrochemical formulations, especially adjuvant surfactants.

The present invention accordingly provides a compound of the formula (l):

R¹ R²N-CH₂-CHO[(AO)ₘ₁ R⁴] -CH₂-OR³ (I)

where
- R¹: is an alkoxylated polyhydroxyhydrocarbyl group; or
- R²: is independently a group as defined for R¹; hydrocarbyl, particularly alkyl; alkoxyalkyl; optionally end-capped alkoxylated hydroxyalkyl; or
- R²: is a group of the formula: -CH₂-CHO[(AO)ₘ₁ R⁴]-CH₂-OR³ where AO, m1, R⁴ and R³ are each independently as defined below;
- R³: is a C₆ to C₃₀ hydrocarbyl, particularly C₈ to C₃₀, more particularly C₁₀ to C₂₀, especially alkyl, alkenyl, alkaryl, aryl or aralkyl;
- each: AO is independently an alkyleneoxy group, particularly a C₂ to C₄ alkyleneoxy group, especially a C₂ or C₃ alkyleneoxy group, or a mixture of C₂ and C₃ alkyleneoxy groups;m1 is from 0 to 50 , but usually at least 0.1, and desirably from 0.5 to 20; and
- R⁴: is hydrogen, or alkyl, particularly C₁ to C₄ alkyl e.g. methyl;
such that the average total number of alkyleneoxy groups in the molecule is at least 3.

The compounds of the formula (I) will usually be made by alkoxylating a compound of the formula (II):

R^{1a}R^{2a}N-CH₂-CHOH-CH₂-OR³ (II)

where
- R^{1a}: is a polyhydroxy hydrocarbyl group;
- R^{2a}: is independently a group as defined for R^{1a}, or is hydrocarbyl, particularly alkyl or alkoxyalkyl or hydroxyalkyl, or is a group of the formula: -CH₂-CHOH-CH₂-OR³ where R³ is as defined for formula (I) above.

Accordingly, the invention includes an alkoxylated compound of the formula (II) in which the average total number of alkyleneoxy groups in the molecule is at least 3. Such compounds can be made by reacting one mole of a compound of the formula (II) with at least 3 moles of an alkylene oxide or a mixture of alkylene oxides.

The compounds of the invention are particularly applicable as agrochemical adjuvants and the invention accordingly includes an agrochemical composition which includes an agrochemically active compound and at least one compound of the formula (I). The invention further includes the use of compounds of any of the formulae (I) as agrochemical adjuvants.

The group R¹ is an alkoxylated polyhydroxy hydrocarbyl, particularly polyhydroxy alkyl, group. Desirably, R¹ is a group of the formula (III):

- R⁵ [-O(AO)ₘ₂-R⁶]ₙ (III)

where
- R⁵: is the residue of a polyhydroxyhydrocarbyl group, which may include substituents, in particular, alkoxy groups e.g. by etherification/acetalisation, of hydroxyl groups not reacted with alkyleneoxy groups, or further polyhydroxy hydrocarbyl group(s);
- each: R⁶ is independently hydrogen, or alkyl, particularly C₁ to C₄ alkyl e.g. methyl;
- AO: is independently as defined above for formula (I);
- each: m2 is independently from 0 to 50 , but usually at least 0.1, and desirably from 0.5 to 20;
- n: is from 2 to 10, particularly from 3 to 6.

The core residue of the group R¹, R⁵ in formula (III), is desirably the residue of a group which includes a linear C₄ to C₇ chain, typically an alkyl chain, with at least two and desirably from 3 to 6 hydroxyl groups directly bonded to chain carbon atoms. In the group R¹ these hydroxyl groups are reacted with alkyleneoxy group(s), usually to give an average degree of polyalkoxylation of at least 0.5, more usually at least 1 per (original) hydroxyl group. The core residue may include substituents, in particular, alkoxy groups e.g. by etherification of further hydroxyl groups or further polyhydroxy hydrocarbyl, e.g. polyhydroxy alkyl, group(s), but the group desirably includes at least three free hydroxyl groups including such hydroxyl groups on substituents of the basic chain, which are available for alkoxylation in producing the compound of the formula (I). Particularly, this core residue is the residue of an open chain tetratol, pentitol, hexitol or heptitol group or an anhydro e.g. cycloether anhydro, derivative of such a group. Especially desirably, this core residue is the residue of, or a residue derived from, a sugar, particularly a monosaccharide such as glucose, fructose or sorbitol, a disaccharide such as maltose or palitose or a higher oligosaccharide. It is particularly convenient that this core residue is the residue of a reducing sugar, because the corresponding amine intermediates in making the compounds of this invention (see amines formula (V) below) can be made by straightforward reductive alkylation reactions with an amine of the formula H₂NR².

In the compounds of the formula (I) of and used in this invention the group R¹ is present as or as part of the hydrophile. Thus it will usually be desirable that the hydrophilicity of this group is not unduly reduced. The open chain form of such groups is typically the most hydrophilic form and will thus usually be the form desired. Groups including internal cyclic ether functionality can however be used, if desired, and may be obtained inadvertently if the synthetic route exposes the group to relatively high temperatures or other conditions which promote such cyclization.

Where this core residue of the group R¹ is the residue of, or a residue derived from, a monosaccharide, the saccharide derived group or residue will usually be present as an open chain material. Where R¹ is the residue of, or a residue derived from, an oligosaccharide it can be considered as an open chain mono-saccharide derived group or residue with a saccharide or oligosaccharide substituent which may be cyclic or a chain of cyclic residues, which in the compounds of the invention will usually be alkoxylated. The saccharide or oligosaccharide group may also carry other substituents such as alkyl, particularly C₈ to C₂₀ alkyl groups or may include acetal/ketal substitution.

Useful R¹ groups are derived from glycoses and are of the formula:

-CH₂ - [CHO(AO)ₘ₃-R⁶]₄ - CH₂O(AO)ₘ₄-R⁶ (IIIa)

where
each AO, each n1, and each R6 is as defined above in formula (III),

In particular R¹ has a core residue corresponding to residues from glucose, mannose or galactose. In this case the group -NR¹ R² is of the formula:

-NR²-CH₂- [CHO(AO)ₘ₃-R⁶]₄- CH₂O(AO)ₘ₄-R⁶ (IIIa')

where
each AO and each R⁶ is as defined above in formula (III), and each m3 and m4 is independently from 0 to 50 , but usually at least 0.1, and desirably from 0.5 to 20, particularly form 1 to 5. The group R¹ is conveniently called an alkoxylated glycamine group. Most commonly the core residue of the group R¹ will be derived from glucose and the corresponding amines may be called alkoxylated glucamines (as they will usually be made from glucose) or alkoxylated sorbitylamines (as they include no aldehyde/enol functionality). Strictly, such compounds are alkoxylated derivatives of 1-deoxy-glycitols (and 1-deoxyglucitols) and can be referred to as alkoxylated 1-deoxyglycitylamines (and 1-deoxyglucitylamines) or as corresponding alkoxylated aminoglycitols (and aminoglucitols).

The group R² can be a group as defined for R¹ or a hydrocarbyl, alkoxyalkyl, optionally end-capped alkoxylated hydroxyalkyl; or a group of the formula: -CH₂-CHO[(AO)ₘ₁R⁴]-CH₂-, where AO, m1 and R⁴ are independently as defined above for formula (I).

Where group R² is hydrocarbyl, it is desirably an alkyl or alkenyl group, and typically it has from 1 to 30, more usually from 1 to 22, carbon atoms. R² can be a blocking group (mainly used to keep the synthesis straightforward), and is then typically a lower e.g. C₁ to C₆, alkyl group, particularly a methyl or ethyl group. R² can be a longer chain e.g. C₆ to C₃₀, particularly a C₈ to C₂₂ alkyl, group and such a longer chain group will tend to act as a secondary hydrophobe in the molecule. R² can also be a substituted alkyl group e.g. an alkoxy substituted alkyl group, particularly a C₁ to C₆ alkyl group substituted with an alkoxy, particularly a C₁ to C₆ alkoxy and especially a methoxy, ethoxy or propoxy, group, so that the alkoxyalkyl group is particularly a 2-methoxyethyl, 2-ethoxyethyl, 3-methoxypropyl, or 3-ethoxypropyl group. R² can also be an aralkyl group, particularly a C₇ to C₁₂ aralkyl group, such as a benzyl group.

R² may also be as defined for R¹ and in this case will usually, but not necessarily be the same as R¹ (allowing for statistical variation in the number of alkyleneoxy groups).

R² may also be an optionally capped polyalkyleneoxy group and in this case is desirably a group of the formula is -(AO)ₘ₅R⁵ where AO is independently as defined for formula (I) above, m5 is from 0 to 50 , but usually at least 0.1, and desirably from 0.5 to 20, and R⁵ is hydrogen, or alkyl, particularly C₁ to C₄ alkyl e.g. methyl. When R² is (poly)alkyleneoxy it is likely that the group will be derived from a hydroxyalkyl particularly a C₂ to C₆ hydroxyalkyl group e.g. 2-hydroxyethyl, or 2- or 3-hydroxypropyl, substituent on the nitrogen atom in the precursor to alkoxylation e.g. a compound of the formula (la).

The group: -CH₂-CH[O(AO)ₘ₁ R⁴]-CH₂- links the hydrophilic substituted amino group with the hydrophobic group R³. Usually it will be based on a glycidyl group derived from an epoxy functional compound, which provides suitable reactivity to enable the "linking" reactions. The basic group includes a hydroxyl group, which will usually carry (poly)alkyleneoxy substitution in the final compound, i.e. m1 >0, and is usually at least 0.1 (see below on the degree of alkoxylation). This linking group is connected to the group R³ by an oxygen atom.

Each alkyleneoxy group AO can be C₂ to C₄ alkyleneoxy, but is desirably ethyleneoxy (-C₂H₄O-) or propyleneoxy (-C₃H₆O-). Polyalkyleneoxy chains in the molecule can be homopolymeric or copolymeric, including particularly mixtures of ethyleneoxy and propyleneoxy residues, which may be in a random (statistical) or block arrangement. Where the molecule includes residues of both ethyleneoxy and propyleneoxy groups the overall molar ratio of ethyleneoxy to propyleneoxy groups is desirably from 1:1 to 10:1.

The overall number of alkyleneoxy residues in the molecule is at least 3 and may be up to about 100 and is in particular from 4 to 50, more usually from 5 to 30, especially from 5 to 20, including any alkyleneoxy residues on oligo-polyhydroxyhydrocarbyl residues in R¹. As is noted above, the compounds of the formula (I) will usually be made by alkoxylating a compound of the formula (Ia) so the distribution of alkyleneoxy units will be determined by the reactivity of the hydroxyl groups in the compound being alkoxylated (secondary hydroxyl groups such as that in the linking group - see above - are less reactive than primary hydroxyl groups), the nature of the alkylene oxide, the proportions of reagents and the reaction conditions used. Where the overall level of polyalkoxylation is relatively low e.g. up to an average of about 1 in each chain, it is possible that chains based on the less reactive hydroxyl groups in the substrate being alkoxylated, may be so short that they are not detectable by analysis of the product, corresponding to an average chain length of or close to zero. However, the length of each (poly)alkyleneoxy chain i.e. the average value of each index m (m1, m2 ; m3, m4 and m5), is usually at least 0.1, more usually at least 0.5 and desirably from 1 to 20 particularly 1 to 10. The number of units in any of the (poly)oxy-alkylene chains, 'm', is an average value and may be non-integral.

The group R³ is a hydrophobic hydrocarbyl group, particularly an alkyl or alkenyl group. R³ may be a straight chain group or may be branched or a mixture of straight chain and branched moieties, and it is a C₆ to C₃₀, usually C₈ to C₃₀, more usually a C₁₀ to C₃₀, particularly a C₁₂ to C₂₀, especially a C₁₂ to C₁₈, group. R³ may also be an alkyl phenyl group e.g. a C₈ to C₁₈ alkyl phenyl group and particularly a 3-linear alkyl phenyl group. Such groups can be derived from cardanols (3-alkyl phenols) which are readily biodegradable compounds.

In particular the invention is directed to the compounds of the formulae (IVa) and (IVb):

R¹R^{2'}N - CH₂ - CH[O(AO)ₘ₁ R⁴] - CH₂ - O - R³ (IVa)

(R¹)₂N - CH₂ - CH[O(AO)ₘ₁ R⁴] - CH₂ - O - R³ (IVb)

where each R¹, R², R³, R⁴, OA, and m1 is independently as defined for formula (I) and R²' is an alkyl, particularly a C₁ to C₄ alkyl, e.g. methyl group.

The compounds of and used in the invention can be made by routes involving generally conventional synthetic steps. In particular, compounds of the formula (I) can be made by alkoxylating a compound of the formula (II) with a suitable alkylene oxide particularly ethylene and/or propylene oxide under alkoxylation conditions, usually in the presence of a basic catalyst.

Compounds of the formula (II) may be made by reacting an amine (IV): R¹ R²NH (IV) with a glycidyl ether (VI): 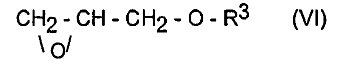 where R¹, R², and R³ are as defined above, under nucleophilic epoxide ring opening conditions. The reaction of an epoxide and an amine in this synthesis can be carried out by heating the reagents in solution or dispersion in an inert solvent or diluent (glycols such as monopropylene glycol are suitably inert for this purpose).

Precursors used above can be made by the following general routes:
Amines of the formula (IV) (R¹R²NH) can be made by reductive alkylation of an amine R²NH with a reactive precursor of the residue R¹, e.g. a reducing sugar of which R¹H is a (possibly notional) 1-deoxy derivative.
Glycidyl ethers of the formula (VI) can be made by reacting alcohols of the formulae R³OH with epi-chlorohydrin under nucleophilic substitution conditions. Ring opening of the epoxy function may be reversed by subsequent treatment with base.

As noted above, the compounds of the formula (I) above can be used in agrochemical formulations as adjuvants. The compounds of the formula (I), particularly those of the formulae (IVa) and (IVb), can be included in formulations with agrochemically active compounds (so-called built-in formulations), which may be formulated as concentrates that are intended for dilution prior to use e.g. by spraying, or may be added to spray formulations shortly before spraying e.g. by addition of the adjuvant or a formulation containing the adjuvant, to the spray tank (as in so-called spray tank addition or mixing).

Accordingly the invention includes agrochemical formulations incorporating compounds of the formula (I), particularly formulae (IVa) and (IVb), as adjuvants. The invention further includes agrochemical formulations, particularly formulated as dilutable concentrates which include at least one agrochemically active compound, and at least one compound of the formula (I), particularly of the formulae (IVa) or (IVb), as adjuvants. The invention also includes the use of compounds of the formula (I), particularly those of the formulae (IVa) and/or (IVb) as agrochemical adjuvants.

The compounds of the invention can be used (particularly as adjuvants) with a wide range of agrochemical active materials and specifically, the active component of the formulation may be one or more plant growth regulators, herbicides, and/or pesticides, for example insecticides, fungicides, acaricides, nematocides, miticides, rodenticides, bactericides, molluscicides and bird repellants. Specific examples of actives include:
Herbicides: including
   water soluble, particularly non-selective, herbicides, more particularly phosphonomethyl glycines (glyphosate). Glyphosate is generally used in the form of one or more of its water soluble salts including without limitation the *iso*-propylammonium, trimethylsulphonium, ammonium or alkali metal such as potassium or sodium, salts. Other examples of water soluble non-selective herbicides include phosphinyl amino acids such as glufosinate {2-amino-4-(hydroxymethylphosphinyl) butanoic acid} particularly as the ammonium salt and bipyridinium compounds such as paraquat {1,1'-dimethyl-4,4'-bipyridinium}; triazines such as atrazine {6-chloro-*N*-ethyl-N-(1-methylethyl)-1,3,5-triazine-2,4-diamine, and Prometryn {N,N'-bis(1-methylethyl)-6-(methylthio)-1,3,5-triazine)-2,4-diamine}; substituted ureas such as Diuron {N'-(3,4-dichlorophenyl)-N,N-dimethylurea}; sulphonyl ureas such as metsulfuron-methyl {2-[[[[(4-methoxy-6-methyl-1,3,5-triazin-2-yl) amino]carbonyl]amino]sulfonyl]benzoate}, triasulfuron {2-(2-chloroethoxy)-N-[[(4-methoxy-6-methyl-1,3,5-triazin-2-yl)amino]carbonyl]benzenesulfonamide}, tribenuron-methyl {methyl 2-[[[[(4-methoxy-6-methyl-1,3,5-triazin-2-yl)-methylamino]carbonyl]amino]sulfonyl] benzoate} and chlorsulfuron {2-chloro-*N*-[[(4-methoxy-6-methyl-1,3,5-triazin-2-yl) amino]carbonyl] benzenesulfonamide}; pyridine carboxylic acids such as clopyralid {3,6-dichloropyridine-2-carboxylic acid}; aryloxy alkanoic acids such as 2,4-D {2,4-dichlorophenoxyacetic acid}; 2-(4-aryloxyphenoxy)propionic acids such as clodinafoppropargyl {prop-2-ynil (R)-2-[4-(5-chloro-3-fluoropyridinr-2-yloxy) phenoxy]-propionate}; and bis-carbamates such as Phenmedipham {3-[(methoxycarbonyl)amino]phenyl (3-methyl phenyl)carbamate}.
Fungicides: including
   thiocarbamates, particularly alkylenebis(dithiocarbamate)s, such as Maneb {[1,2-ethanediyl-bis-[carbamodithiato] (2-)] manganese} and Mancozeb {[[1,2-ethanediylbis[carbamodithiato]](2-)]manganese mixture with [[1,2-ethanediylbis[carbamodithiato]] (2-)]zinc}; strobilurins such as azoxystrobin {methyl (E)-2-[[6-(2-cyanophenoxy)-4-pyrimidinyl]oxy]-a-(methoxymethylene)benzeneacetate} and kresoxim-methyl {(E)-a-(methoxyimino)-2-[(2-methylphenoxy)methyl]benzeneacetic acid methyl ester}; dicarboximides such as Iprodione {3-(3,5-dichlorophenyl)-N-isopropyl-2,4-dioxo imidazolidine-1-carboxamide}; halogenated phthalonitriles such as 2,4,5,6-tetrachloro-1,3-dicyanobenzene; benzimidazoles such as Carbendazym {methyl benzimidazol-2-yl carbamate}; azoles such as Propiconazole {1-[2-(2,4-dichlorophenyl)-4-propyl-1,3-dioxolan-2-yl-methyl-1H-1,2,4-triazole}, and Tebuconazole {(RS)-1-p-chlorophenyl-4,4-dimethyl-3-(1H-1,2,4-triazole-1-ylmethyl)-pentan-3-ol}; and inorganic fungicides such as Copper hydroxide {Cu(OH)2}; benzoyl ureas such as Diflubenzuron {N-[[(4-chlorophenyl)amino]carbonyl]-2,6-difluorobenzamide)} and pyrethroid insecticides; and
Acaricides including: tetrazines such as Clofentezine {3,6-bis(2-chlorophenyl)-1,2,4,5-tetrazine}.

Among water soluble active materials particularly suitable actives include, non-selective herbicides, particularly *N*-(phosphonomethyl) glycine type herbicides, such as glyphosate, and phosphinyl amino acids, such as Glufosinate, particularly as the ammonium salt. Such water soluble actives can be used as the sole active in for example in aqueous solutions or in water dispersible granules or other solid formulations, but may also be used in combination with water insoluble or immiscible actives in multi active formulations. In particular, formulations can be made up using a water soluble (non-specific) herbicide such as glyphosate and/or Glufosinate, with a selective herbicide, such as a sulphonyl urea e.g. metsulfuron-methyl, pyridine carboxylic acid e.g. clopyralid, aryloxy alkanoic acids e.g. 2,4-D, substituted ureas e.g. diuron, or 2-(4-aryloxyphenoxy) propionic acids e.g. clodinafoppropargyl, and/or with an insecticide and/or fungicide. Numerous formulations comprising glyphosate in combination with other active ingredients have been published and will be familiar to those skilled in the art.

Generally, in use as adjuvants in agrochemical formulations, the compounds of and used in this invention can be added to agrochemical formulations as part of the tank mix (the formulation actually used for spraying) or can be included in pre-formulated products which usually take the form of concentrates (including solution concentrates and suspension concentrates), emulsifiable concentrates or solid formulations such as dispersible granules.

When added to tank mix compositions for spray formulations using current spray application rates, generally from 100 to 400 I(spray).ha⁻¹ (crop treated), usually about 300 l.ha⁻¹, the concentration of the active agrochemical is typically from about 0.05 to about 3%, more usually from 0.1 to about 0.5 and particularly about 0.2 % by weight of the spray formulation and the concentration of adjuvant will typically be 0.02 to about 2%, more usually 0.2 to about 1% and particularly about 0.1%. The weight ratio of active agrochemical to adjuvant is usually from 1:5 to 10:1, more usually from 1:2 to about 4:1. These figures correspond to crop application rates of the active agrochemical generally in the range 300 to 4000 g.ha⁻¹, more usually from 750 to about 2000 g.ha⁻¹ (the actual amount depending on the particular crop, agrochemical and effect desired). For low volume spraying, generally higher spray concentrations will be used, but the ratio of agrochemical to adjuvant will be within the ranges given above.

The surfactants of the formula (I) can be used as "built in" adjuvants in concentrate agrochemical formulations that are intended for dilution prior to use. In such concentrates, the concentration of active agrochemical is typically from about 5 to about 60%, more usually from 10 to 40% and the adjuvant concentration is from about 3 to about 50%, more usually from 5 to 30% by weight of the concentrate. The use as built in adjuvants in concentrates is particularly applicable for concentrates where the carrier is aqueous and the active is or includes one or more water soluble herbicides, such as glyphosate and glufosinate.

As adjuvants the compounds of and used in this invention can provide faster effectiveness of agrochemicals especially water soluble herbicides, particularly of the glyphosate type, and can have significantly lower toxicity, particularly aquatic toxicity, than conventional adjuvants, particularly those based on fatty amine ethoxylates. The improved toxicity is also important when the compounds are used to provide other surfactant effects in agrochemical formulations..

Agrochemical formulations of the invention can be made up using surfactants of the formula (I) in a variety of formulation types including:
i Water soluble liquids (aqueous dilutable solutions) in which water soluble agrochemical active(s) and surfactant(s) are dissolved in water and the formulation is diluted with water before use. In such formulations the surfactant(s) are usually present as adjuvants or wetting agents. Typically such formulations use concentrations within the ranges:

| | |
|---|---|
| agrochemical active : | 100 to 500 g.l⁻¹ |
| surfactant : | 30 to 500 g.l⁻¹ |

The surfactant can be a mixture of compounds of the formula (I) and other, particularly non-ionic surfactants (see also below about mixtures).
Possible other components in such formulations include
i antifoams, particularly polysiloxane antifoams, typically included at a concentration of from 0.1 and 10% by weight of the concentrate formulation; and
ii viscosity modifiers : gums, e.g. xanthan gums, modified cellulose e.g. carboxy- methyl, -ethyl or -propyl cellulose, typically included at between 0.01 and 5% by weight of the concentrate formulation.
   Such concentrate formulations can be made by simple mixing of the components.
   Conveniently this may be carried out by dissolving the agrochemical active(s) and the adjuvant surfactant(s) and any other components in water to give either a concentrate for subsequent dilution to end use concentrations or directly at end use concentration e.g. in the spray tank.

ii Liquid concentrates, including emulsifiable concentrates, can include compounds of the formula (I). In liquid concentrates the surfactants are typically present as adjuvants, wetting agents, emulsifiers or solubilisers. The amount of surfactant(s) used in such concentrates is typically from 1 to 30% by weight of the concentrate. Other surfactants such as non-ionic, amphoteric, cationic or anionic or combinations of such surfactants may be used together with compounds of the formula (I) (see also below about mixtures). In liquid concentrates, typically use concentrations are within the ranges:

| | |
|---|---|
| agrochemical active : | 0.2 to 10% by weight (though with liquid agrochemicals, the concentration can be up to 90%); and |
| surfactant : | 1 to 20% by weight of the liquid concentrate. |

Liquid concentrate agrochemical formulations may also include:
solvents such as monoethylene glycol, diethylene glycol, glycerol, (mono)propylene glycol, which, especially with propylene glycol, may also act as a humectant, typically in an amount from 5 to 500% by weight of the surfactants;
oils, particularly vegetable or mineral oils, such as spray oils, typically in an amount from 5 to 500% by weight of the surfactants;
salts, such as ammonium chloride and/or sodium benzoate, and/or urea as gel inhibition aids typically in an amount from 1 to 10% by weight of the formulation.
iii Solid dispersible granules - the surfactant will usually be included as an adjuvant or a dispersing agent and can be included in a granular agrochemical active formulation or itself be formulated as dispersible granules. Typically granules including agrochemical active contain from 1 to 80%, more usually from 1 to 30%, by weight of the granule of active. When included in granules containing an agrochemical active, the adjuvant typically forms from 5 to 50% by weight of the granule.

The granules can include clathrates, particularly urea clathrates, in particular incorporating the surfactant, especially as an adjuvant. Such clathrates can be made by forming a comelt, including the urea and surfactant, and cooling by e.g. spray cooling. Such clathrate solid granules will typically have a ratio of urea to surfactant adjuvant of from 1:2 to 5:1 by weight. Clathrates can be included in the agrochemical granules or and desirably formulated as a separate adjuvant granule which can be used by direct mixing with granular agrochemical active compositions.

When the adjuvant is provided in separate granules from the active agrochemical, the mixing rate of adjuvant granules to agrochemical active granules will depend on the respective concentrations in the granules, but will usually be such as to give a ratio of adjuvant to agrochemical active within the ranges described above.

In such granular formulations, other possible components of the granules include:
binders, particularly binders which are readily water soluble to give low viscosity solutions at high binder concentrations, such as polyvinylpyrrolidone, polyvinylalcohol, carboxymethyl cellulose, gum arabic, sugars, starch, sucrose and alginates;
diluents, absorbents or carriers such as carbon black, talc, diatomaceous earth, kaolin, aluminium, calcium and/or magnesium stearate, sodium tripolyphosphate, sodium tetraborate, sodium sulphate, sodium, aluminium or mixed sodium-aluminium silicates; and sodium benzoate;
disintegration agents, such as surfactants, materials that swell in water, for example carboxymethyl cellulose, collodion, polyvinyl pyrrolidone and/or microcrystalline cellulose swelling agents; salts such as sodium and/or potassium acetate, sodium carbonate, bicarbonate and/or sesquicarbonate, ammonium sulphate and/or dipotassium hydrogen phosphate;
wetting agents such as alcohol alkoxylates, particularly ethoxylates or ethoxylate/propoxylates;
dispersants such as sulphonated naphthalene formaldehyde condensates and acrylic copolymers; and
antifoam agents, typically at a concentration of from 1 to 10 % by weight of the granule.

Spray formulations at application concentration, including surfactants of the formula (I), particularly as adjuvants, can be made up by diluting/dispersing the agrochemical active and the adjuvant in the spray liquid (usually water). Also concentrate forms of the agrochemical formulation can be used, for example:
i liquid concentrate containing the agrochemical active and, particularly adjuvant, surfactant dissolved in water;
ii liquid concentrate containing the agrochemical active dissolved or dispersed in a non-aqueous, water immiscible liquid, which may be an emulsifiable concentrate and may include a proportion of water, including an adjuvant surfactant;
iii liquid concentrate containing the agrochemical active dissolved or dispersed in a non-aqueous, water miscible liquid and including an adjuvant surfactant;
iv a solid granular concentrate of or containing the agrochemical active and optionally including an adjuvant surfactant, or the adjuvant surfactant can be provided separately for example as a solution in a solvent (water or a non-aqueous solvent) or a granule, particularly a urea adduct, containing the adjuvant.

Concentrated forms of the agrochemical active will typically be diluted from 10 to 10000, particularly 30 to 1000 times to generate the agrochemical spray for use.

Agrochemical formulations often include more than one surfactant either because surfactants are used in combination to achieve the desired effect or used to provide different effects. It is thus possible in this invention to use combinations of more than one surfactant of the formula (I) or to combine surfactant(s) of the formula (I) with other surfactants.

In optimising adjuvancy, mixtures of adjuvant surfactants can be used and the invention includes agrochemical formulations including compounds of the formula (I) in combination with other adjuvant materials. Commonly such other adjuvants may be non-ionic surfactant adjuvants and examples include so-called hydrocarbyl, particularly alkyl, polysaccharides (generally more correctly described as oligosaccharides) particularly alkyl polyglycosides; hydrocarbyl, particularly alkyl, amine alkoxylates, particularly ethoxylates, linear or mono-branched alcohol alkoxylates, particularly ethoxylates; sorbitol fatty acid esters; sorbitan fatty acid esters; and ethoxylated sorbitan fatty acid esters. The proportion of compounds of the formula (I) and other adjuvants, particularly non-ionic surfactant adjuvant, (when used) is typically from 1:5 to 10:1, more usually from 1:1 to 5:1 by weight. The proportions and concentrations of adjuvants referred to above include both compound(s) of the formula (I) and other, particularly non-ionic surfactant adjuvants.

Co-adjuvants, including ionic and/or inorganic materials, for example ammonium sulphate, may be included in adjuvant containing agrochemical formulations of the invention, particularly with non-ionic surfactant adjuvants, especially including those of the formula (I), optionally used in combination with other, particularly non-ionic, surfactant adjuvants.

Generally when other surfactants, especially non-ionic surfactants are used, the compound(s) of the formula (I) will be at least 25% and more usually at least 50% of the total surfactant used to provide the desired effect.

Other conventional components can be included in such formulations such as one or more oils e.g. mineral oil(s), vegetable oil(s) and alkylated vegetable oil(s) which are, typically C₁ to C₈, alkyl mono esters of vegetable oil fatty acids; solvents and/or diluents such as ethylene and/or propylene glycol or low molecular weight alcohols, which act to solubilise the formulation and/or to reduce the viscosity and/or to avoid or reduce dilution problems e.g. the formation of gels. In particular where non-aqueous, particularly those which are not miscible with or soluble in water, liquids are included e.g. as solvents for the agrochemical and/or in a concentrate to form an emulsion on dilution with water for spraying, other surfactants may be included as solubilisers and/or emulsifiers. Such materials will typically be chosen from anionic, cationic and/or non-ionic surfactants for their effectiveness in solubilisation and or emulsification. Such other surfactant components will, as with formulations using purely conventional surfactants, be used in amounts based on the desired effect.

Other surfactants may also be included to improve wetting. Examples of such wetting agents include nonionic surfactants such as alcohol ethoxylates for example of Cg to C₁₅, particularly primary, alcohols, which may be linear or branched, particularly mono-branched, with from 5 to 30 moles of ethylene oxide; and alkoxylates of such alcohols particularly mixed ethoxylate/propoxylates which may be block or random mixed alkoxylates, typically containing from 3 to 10 ethylene oxide residues and from 1 to 5 propylene oxide residues, particularly where
the polyalkoxylate chain is terminated with propylene oxide unit(s); polyoxyethylene/polyoxypropylene copolymers, particularly block copolymers, such as the Synperonic PE series of copolymers available from Uniqema, and alkyl polysaccharides; anionic surfactants e.g. isethionates, such as sodium cocoyl isethionate, naphthalene sulphonic acids or sulphosuccinates. The amounts of wetting surfactants are typically similar to or the same as the levels typically used to provide adjuvant effects (see above).

The compounds of the formula (I) may be used in combination with non-surfactant materials, particularly solvents or solvation aids such as glycols such as monopropylene glycol and/or polyethylene glycol. The proportion of compounds of the formula (I) to such solvents or solvation aids, (when used) is typically from 1:5 to 10:1, more usually from 1:1 to 5:1 by weight.

The invention includes a method of treating vegetation by applying to plants and/or soil a composition including a surfactant of the formula (I) and an agrochemical according to the invention. The agrochemical may be one or more of the types of actives described above, particularly, one or more growth regulators, herbicides, and/or pesticides, for example insecticides, fungicides or acaricides. This method of the invention includes:
(i) a method of killing or inhibiting vegetation by applying a formulation which includes one or more growth regulators and/or herbicides and at least one compound of the general formula (I) as an adjuvant, and/or
(ii) a method of killing or inhibiting plant pests by applying a formulation which includes one or more pesticides, for example insecticides, fungicides or acaricides, and at least one compound of the general formula (I) as an adjuvant.

Other additives can be included in agrochemical formulations of the invention including:
inorganic salts such as ammonium chloride, calcium chloride and/or sodium benzoate and/or urea in an amount of from 0.01 to 1% by weight of composition.
antifoams which can be silicon based materials such as organopolysiloxanes, which are typically used in an amount from 0.1 to 10%, preferably 0.2 to 6% by weight of the surfactant; 0.01 to 5%, particularly 0.02 to 2% by weight of agrochemical concentrate and 0.0001 to 0.1 % preferably 0.001 to 0.05% by weight of a spray formulation at end use dilution;
viscosity modifiers, particularly gums such as xanthan gums; cellulose derivatives, such as carboxyl-methyl, -ethyl, or -propyl cellulose, typically used at from 0.01 to 5 wt % of a concentrated formulation; and
other non surfactant materials such as stabilisers and/or anti-microbials, typically used at from 0.01 to 5 wt % of a concentrated formulation.

The following Examples illustrate the invention. All parts and percentages are by weight unless otherwise stated.

### Materials

| | |
|---|---|
| N-methylglucamine (NMG) | *N*-methyl-*N*-(1-deoxyglucityl) amine |
| GE1 | C8/10 alkyl glycidyl ether, 89% active |
| GE2 | C13/15 alkyl glycidyl ether, 89% active |

Synthesis Examples SE1 to SE6 illustrate the synthesis of the compounds of the formula (I).

### Synthesis Examples SE1

Random 8-ethoxylate-2-propoxylate of *N*-methyl glucamine C₈/C₁₀ glycidyl ether

N-methyl glucamine (260.3 g, 1.33 mol) was added slowly (in aliquots to control the exotherm) to glycidyl ether GE1 (300 g, (equivalent to 267 g of 100% material), 1.33 mol) in a 1 litre flanged flask at 115°C. The temperature rapidly increased to 140°C even with the thermostatting effect of the oil heating bath used. The reaction mixture became completely clear at about 120°C. After addition, the temperature was reduced to 100°C and the mixture stirred at this temperature for a further 30 minutes. The mixture was then cooled and he N-methyl glucamine glycidyl ether product was removed from the flask.

229 g (0.58 mol) *N*-methyl glucamine C₈/C₁₀ glycidyl ether from the previous stage was charged to an alkoxylation reactor at 80°C with 1.2g KOH pellets as catalyst, the mixture was deaerated under vacuum at 100°C and then heated to 125°C. 271 g (5.78 mol; 204 g EO and 67 g PO) of a 4:1 molar mixture of ethylene oxide and propylene oxide were then added and allowed to react to give the 494 g (98.7% of theory) of the alkoxylated product which was discharged from the reactor. This product had a hydroxyl value of 399 mg(KOH).g⁻¹ and an acid value of 0.8 mg(KOH).g⁻¹.

### Synthesis Examples SE2

Random 16-ethoxylate-4-propoxylate of *N*-methyl glucamine C₈/C₁₀ glycidyl ether

The title compound was made by the general method described of Synthesis Example 1 but using proportionately twice the amount of the mixed alkylene oxides in the alkoxylation stage. The yield was 98.2% of theory and the product had a hydroxyl value of 281 mg(KOH).g⁻¹ and an acid value of 0.4 mgKOH.g-1.

### Synthesis Examples SE3

Random 32-ethoxylate-8-propoxylate of N-methyl glucamine C₈/C₁₀ glycidyl ether The title compound was made by further alkoxylating a portion of the product of Synthesis Example 2 using an additional amount of the mixed alkylene oxides in the alkoxylation stage equal to the amount used in SE2. The yield was 97.8% of theory and the product had a hydroxyl value of 158 mg(KOH).g⁻¹ and an acid value of 0.4 mg(KOH).g⁻¹.

### Synthesis Examples SE4

Random 8-ethoxylate-2-propoxylate of *N*-methyl glucamine C₁₃/C₁₅ glycidyl ether N-methyl glucamine (220.3 g, 1.13 mol) was added slowly (in aliquots to control exotherm) to glycidyl ether GE2 (339 g, (equivalent to 301.7 g of 100% material), 1.12 mol) in a 1 litre flanged flask at 115°C. The heater for the flask was isolated during addition, during which the temperature rose to ca 130°C. After 10 minutes the reaction mixture became clear and was one phase; the reaction was continued for a further 1 hour at 100°C with stirring. The N-methyl glucamine glycidyl ether intermediate product was then cooled and removed from the flask.

This intermediate *N*-methyl glucamine C₁₃/C₁₅ glycidyl ether was alkoxylated as described in Synthesis Example 1. The alkoxylated product was obtained in a yield of 99.5% of theory and had a hydroxyl value of 376 mg(KOH).g⁻¹ and an acid value of 0.3 mg(KOH).g⁻¹.

### Synthesis Examples SE5

Random 16-ethoxylate-4-propoxylate of *N*-methyl glucamine C₁₃/C₁₅ glycidyl ether The title compound was made by the general method of Synthesis Example SE2, but using the intermediate N-methyl glucamine C₁₃/C₁₅ glycidyl ether made as described in Synthesis Example 4. The title compound was obtained in a yield of 97.7% of theory and had a hydroxyl value of 304 mg(KOH).g⁻¹ and an acid value of 0.4 mg(KOH).g⁻¹.

### Synthesis Examples SE6

Random 32-ethoxylate-8-propoxylate of *N*-methyl glucamine C₁₃/C₁₅ glycidyl ether The title compound was made by the general method of Synthesis Example SE3, but using the intermediate *N*-methyl glucamine C₁₃/C₁₅ glycidyl ether made as described in Synthesis Example 4. The title compound was obtained in a yield of 98.9% of theory and had a hydroxyl value of 289 mg(KOH).g⁻¹ and an acid value of 0.2 mg(KOH).g⁻¹.

Application Examples AE1 and AE2 illustrate the use of compounds of the formula (I) as agrochemical adjuvants.

### Test Species

| | |
|---|---|
| ECHCG | *Echinochloa crus-galli* |
| SORHA | *Sorghum halepense* |
| ABUTH | *Abutilon Theophrasti* |
| POROC | *Portulaca oleracea* |
| SIDSP | *Sida Spinosa* |
| SOLNI | *Solanum nigra* |

### Application Example AE1

The compound of Synthesis Example SE1 was tested as an adjuvant with glyphosate potassium salt, mixing the glyphosate and adjuvant in a tank mix using concentrations of glyphosate from 0.125 to 2 g.l-1 and of adjuvant from 0.003125 to 0.2 % v/v. The tank mixes were sprayed as a herbicide on a variety of test species at an application rate of 200 I.ha⁻¹ to give application rates of glyphosate of from 25 to 400 g (active ingredient).ha⁻¹. The plants were grown in a warm glasshouse and assessed 19 days after treatment for % control - the mean of three replicates for visually assessed % weed control, relative to unsprayed plants of each species in the same test. The results are set out in Table 1 below.

**Table 1**

| Glyphosate (g ai.ha⁻¹) | Adjuvant (% v/v) | Grasses | | Broad leaved species | | | |
|---|---|---|---|---|---|---|---|
| | | ECHCG | SORHA | ABUTH | POROC | SIDSP | SOLNI |
| 25 | 0 | 21 | 8 | - | - | - | - |
| | 0.003125 | 15 | 17 | - | - | - | - |
| | 0.0125 | 10 | 7 | - | - | - | - |
| | 0.05 | 22 | 8 | - | - | - | - |
| | 0.2 | 37 | 45 | - | - | - | - |
| 50 | 0 | 47 | 47 | - | 3 | 30 | 37 |
| | 0.003125 | 48 | 40 | 33 | 7 | 30 | 37 |
| | 0.0125 | 47 | 22 | 33 | 4 | 38 | 35 |
| | 0.05 | 58 | 20 | 38 | 12 | 42 | 48 |
| | 0.2 | 70 | 84 | 27 | 30 | 31 | 50 |
| 100 | 0 | 67 | 79 | - | 10 | 35 | 65 |
| | 0.003125 | 85 | 90 | 53 | 8 | 33 | 62 |
| | 0.0125 | 82 | 81 | 35 | 9 | 48 | 68 |
| | 0.05 | 84 | 72 | 43 | 18 | 48 | 68 |
| | 0.2 | 89 | 92 | 42 | 37 | 55 | 77 |
| 200 | 0 | 82 | 82 | 53 | 22 | 58 | 77 |
| | 0.003125 | 92 | 93 | 62 | 23 | 63 | 80 |
| | 0.0125 | 90 | 91 | 66 | 20 | 48 | 81 |
| | 0.05 | 93 | 91 | 66 | 45 | 72 | 81 |
| | 0.2 | 97 | 96 | 70 | 64 | 79 | 79 |
| 400 | 0 | - | - | 73 | 38 | 75 | 78 |
| | 0.003125 | - | - | 81 | 42 | 79 | 80 |
| | 0.0125 | - | - | 82 | 43 | 73 | 81 |
| | 0.05 | - | - | 79 | 60 | 83 | 79 |
| | 0.2 | - | - | 83 | 73 | 78 | 82 |

### Application Example 2

Example AE1 was repeated but using the compound made in Synthesis Example SE4 as the adjuvant. The results are set out in Table 2 below.

**Table 2**

| Glyphosate (g ai.ha⁻¹) | Adjuvant (% v/v) | Grasses | | Broad leaved species | | | |
|---|---|---|---|---|---|---|---|
| | | ECHCG | SORHA | ABUTH | POROC | SIDSP | SOLNI |
| 25 | 0 | 21 | 8 | - | - | - | - |
| | 0.003125 | 12 | 8 | - | - | - | - |
| | 0.0125 | 15 | 8 | - | - | - | - |
| | 0.05 | 51 | 32 | - | - | - | - |
| | 0.2 | 53 | 84 | - | - | - | - |
| 50 | 0 | 47 | 47 | - | 3 | 30 | 37 |
| | 0.003125 | 32 | 23 | 40 | 7 | 30 | 37 |
| | 0.0125 | 35 | 20 | 48 | 2 | 38 | 32 |
| | 0.05 | 68 | 68 | 25 | 13 | 38 | 63 |
| | 0.2 | 91 | 89 | 43 | 35 | 38 | 73 |
| 100 | 0 | 67 | 79 | - | 10 | 35 | 65 |
| | 0.003125 | 78 | 78 | 42 | 8 | 37 | 65 |
| | 0.0125 | 83 | 70 | 33 | 18 | 38 | 68 |
| | 0.05 | 90 | 90 | 50 | 43 | 52 | 71 |
| | 0.2 | 98 | 95 | 52 | 46 | 62 | 78 |
| 200 | 0 | 82 | 82 | 53 | 22 | 58 | 77 |
| | 0.003125 | 90 | 87 | 42 | 28 | 50 | 78 |
| | 0.0125 | 89 | 89 | 65 | 36 | 63 | 73 |
| | 0.05 | 95 | 96 | 58 | 45 | 77 | 80 |
| | 0.2 | 98 | 95 | 68 | 50 | 76 | 82 |
| 400 | 0 | - | - | 73 | 38 | 75 | 78 |
| | 0.003125 | - | - | 78 | 42 | 76 | 83 |
| | 0.0125 | - | - | 80 | 47 | 78 | 83 |
| | 0.05 | - | - | 81 | 63 | 78 | 84 |
| | 0.2 | - | - | 85 | 81 | 77 | 85 |

## Claims

1. A compound of the formula (I):
R¹ R²N-CH₂-CHO[(AO)ₘ₁ R⁴] -CH₂-OR³ (I)
where
R¹ is an alkoxylated polyhydroxy hydrocarbyl group; or
R² is independently a group as defined for R¹; hydrocarbyl, particularly alkyl; alkoxyalkyl; optionally end-capped alkoxylated hydroxyalkyl; or
R² is a group of the formula: -CH₂-CHO[(AO)ₘ₁ R⁴]-CH₂-OR³ where AO, m1, R⁴ and R³ are each independently as defined below;
R³ is C₆ to C₃₀ hydrocarbyl, particularly C₈ to C₃₀, more particularly C₁₀ to C₂₀, especially alkyl, alkenyl, alkaryl, aryl or aralkyl;
each AO is independently an alkyleneoxy group, particularly a C₂ to C₄ alkyleneoxy group, especially a C₂ or C₃ alkyleneoxy group, or a mixture of C₂ and C₃ alkyleneoxy groups;
m1 is from 0 to 50 , but usually at least 0.1, and desirably from 0.5 to 20; and
R⁴ is hydrogen, or alkyl;
such that the average total number of alkyleneoxy groups in the molecule is at least 3.

2. An alkoxylated compound of the formula (II)
R^{1a}R^{2a}N-CH₂-CHOH-CH₂-OR³ (II)
where
R^{1a} is a polyhydroxy hydrocarbyl group;
R^{2a} is independently a group as defined for R^{1a}, or is hydrocarbyl, particularly alkyl or alkoxyalkyl or hydroxyalkyl, or is a group of the formula: -CH₂-CHOH-CH₂-OR³ where R³ is a C₆ to C₃₀ hydrocarbyl,
in which the average total number of alkyleneoxy groups in the molecule is at least 3.

3. A compound as claimed in claim 1 wherein R¹ is a group of the formula (III):
- R⁵ [-O(AO)ₘ₃-R⁶]ₙ (III)
where
R⁵ is the residue of a hydrocarbyl group;
R⁶ is hydrogen, or alkyl;
AO is an alkyleneoxy group;
m3 is an average value of from 1 to 20; and
n is from 3 to 10.

4. A compound as claimed in claim 3 wherein R⁵ is the residue (notionally obtained by removing hydroxyl groups from the parent group) of a polyhydroxy alkyl group having a linear C₄ to C₇ chain.

5. A compound as claimed in either claim 3 or claim 4 wherein n is from 3 to 6

6. A compound as claimed in any one of claims 1 or 2 to 4 wherein R² is an alkyl group and R³ is a C₁₀ to C₃₀ alkyl, alkenyl, alkaryl, aryl or aralkyl group.

7. A compound as claimed in any one of claims 1 or 2 to 5 wherein the alkyleneoxy group(s) AO is(are) ethyleneoxy, propyleneoxy or mixtures of ethyleneoxy and propyleneoxy groups.

8. A compound as claimed in claim 6 wherein the total number of alkyleneoxy groups in the compound of the formula (I) is from 5 to 30.

9. An agrochemical composition including as adjuvant a compound of the formula (I) as claimed in any one of claims 1 to 8.

10. A composition as claimed in claims 9 wherein the agrochemically active compound is one or more plant growth regulators, herbicides, and/or pesticides, for example insecticides, fungicides, acaricides, nematocides, miticides, rodenticides, bactericides, molluscicides and/or bird repellants.

11. A composition as claimed in claim 9 wherein the agrochemically active compound is or includes at least one water soluble herbicide.

12. 2 A composition as claimed in claim 10 wherein the water soluble herbicide is or includes at least one phosphonomethyl glycine; at least one phosphinyl amino acid; and/or at least one bipyridinium compound.

13. A composition as claimed in any one of claims 9 to 12 which additionally includes at least one further surfactant.

14. A composition as claimed in claim 13 which additionally includes at least one alkylpolysaccharide surfactant.

15. A method of treating vegetation by applying to plants and/or soil a composition as claimed in any one of claims 9 to 14.

16. A method of killing or inhibiting vegetation by applying a formulation as claimed in any one of claims 9 to 14 which includes one or more growth regulators and/or herbicides and at least one compound of the general formula (I) as defined in any one of claims 1 to 6 as an adjuvant.

17. A method of killing plant pests by applying a formulation as claimed in any one of claims 9 to 14 which includes one or more pesticides, fungicides or acaricides, and at least one compound of the general formula (I) as defined in any one of claims 1 to 6 as an adjuvant.

## Patentansprüche

1. Verbindung der Formel (I):
R¹R²N-CH₂-CHO[(AO)ₘ₁R⁴]-CH₂-OR³ (I)
wobei
R¹ eine alkoxylierte Polyhydroxykohlenwasserstoffgruppe ist; oder
R² unabhängig eine Gruppe wie für R¹ definiert; ein Kohlenwasserstoff, insbesondere ein Alkyl; ein Alkoxyalkyl; ein optional am Ende verkapptes alkoxyliertes Hydroxyalkyl ist; oder
R² eine Gruppe der Formel: -CH₂-CHO[(AO)m₁R⁴]-CH₂-OR³ ist, wobei AO, m1, R⁴ und R³ jeweils unabhängig wie im Folgenden definiert sind;
R³ ein C₆ bis C₃₀-Kohlenwasserstoff, insbesondere C₈ bis C₃₀, genauer C₁₀ bis C₂₀, insbesondere Alkyl, Alkenyl, Alkaryl, Aryl oder Aralkyl ist;
jedes AO unabhängig eine Alkylenoxygruppe, insbesondere eine C₂- bis C₄-Alkylenoxygruppe, speziell eine C₂- oder C₃-Alkylenoxygruppe, oder eine Mischung von C₂- und C₃-Alkylenoxygruppen ist;
m1 von 0 bis 50, aber gewöhnlich wenigstens 0,1, und erwünscht von 0,5 bis 20 ist; und
R⁴ Wasserstoff oder Alkyl ist; so dass die durchschnittliche Gesamtanzahl der Alkylenoxygruppen in dem Molekül wenigstens 3 ist.

2. Alkoxylierte Verbindung der Formel (II)
R^{1a}R^{2a}N-CH2-CHOH-CH₂-OR³ (II)
wobei
R^{1a} eine Polyhydroxy-Kohlenwasserstoffgruppe ist;
R^{2a} unabhängig eine Gruppe wie für R^{1a} definiert ist, oder ein Kohlenwasserstoff, insbesondere ein Alkyl oder ein Alkoxyalkyl oder ein Hydroxyalkyl ist, oder eine Gruppe der Formel: -CH₂-CHOH-CH₂-OR³ ist, wobei R³ ein C₆- bis C₃₀-Kohlenwasserstoff ist,
in welcher die durchschnittliche Gesamtanzahl der Alkylenoxygruppen in dem Molekül wenigstens 3 ist.

3. Verbindung nach Anspruch 1, wobei R¹ eine Gruppe der Formel (III) ist:
-R⁵[-O(AO)ₘ₃-R⁶]ₙ (III)
wobei
R⁵ der Rest einer Kohlenwasserstoffgruppe ist;
R⁶ Wasserstoff oder Alkyl ist;
AO eine Alkylenoxygruppe ist;
m3 ein durchschnittlicher Wert von 1 bis 20 ist; und
n von 3 bis 10 ist.

4. Verbindung nach Anspruch 3, wobei R⁵ der Rest (theoretisch erhalten durch Entfernung von Hydroxylgruppen von der Elterngruppe) einer Polyhydroxyalkylgruppe mit einer unverzweigten C₄- bis C₇-Kette ist.

5. Verbindung entweder nach Anspruch 3 oder nach Anspruch 4, wobei n von 3 bis 6 ist.

6. Verbindung nach einem der Ansprüche 1 oder 2 bis 4, wobei R² eine Alkylgruppe und R³ eine C₁₀- bis C₃₀-Alkyl-, Alkenyl-, Alkaryl-, Aryl- oder Aralkylgruppe ist.

7. Verbindung nach einem der Ansprüche 1 oder 2 bis 5, wobei die Alkylenoxygruppe(n) AO Ethylenoxy-, Propylenoxy- oder Mischungen von Ethylenoxy- und Propylenoxygruppe(n) ist (sind).

8. Verbindung nach Anspruch 6, wobei die Gesamtanzahl der Alkylenoxygruppen in der Verbindung der Formel (I) von 5 bis 30 ist.

9. Agrochemische Zusammensetzung, die eine Verbindung der Formel (I) nach einem der Ansprüche 1 bis 8 als einen Zusatz enthält.

10. Zusammensetzung nach Anspruch 9, wobei der agrochemische Wirkstoff ein oder mehrere Pflanzenwachstumsregulatoren, Herbizide und/oder Pestizide, zum Beispiel Insektizide, Fungizide, Acarizide, Nematozide, Mitizide, Rodentizide, Bakterizide, Molluskizide und/oder Vögel abschreckende Mittel ist.

11. Zusammensetzung nach Anspruch 9, wobei die agrochemisch aktive Verbindung wenigstens ein wasserlösliches Herbizid ist oder enthält.

12. Zusammensetzung nach Anspruch 10, wobei das wasserlösliche Herbizid wenigstens ein Phosphonomethylglycin; wenigstens eine Phosphinylaminosäure; und/oder wenigstens eine Bipyridinverbindung ist oder enthält.

13. Zusammensetzung nach einem der Ansprüche 9 bis 12, welche zusätzlich wenigstens einen weiteren oberflächenaktiven Stoff enthält.

14. Zusammensetzung nach Anspruch 13, welche zusätzlich wenigstens einer Alkylpolysaccharid-oberflächenaktiven Stoff enthält.

15. Verfahren zur Behandlung von Vegetation durch Aufbringen einer Zusammensetzung nach einem der Ansprüche 9 bis 14 auf Pflanzen und/oder den Boden.

16. Verfahren zur Tötung oder Hemmung von Vegetation durch Aufbringen einer Formulierung nach einem der Ansprüche 9 bis 14, welche einen oder mehrere Wachstumsregulatoren und/oder Herbizide und wenigstens eine Verbindung der allgemeinen Formel (I), wie in einem der Ansprüche 1 bis 6 definiert, als einen Zusatz enthält.

17. Verfahren zur Tötung von Pflanzenschädlingen durch Aufbringen einer Formulierung nach einem der Ansprüche 9 bis 14, welche wenigstens ein oder mehrere Pestizide, Fungizide oder Acarizide und wenigstens eine Verbindung der allgemeinen Formel (I), wie in einem der Ansprüche 1 bis 6 definiert, als einen Zusatz enthält.

## Revendications

1. Composé de formule (I) :
R¹R²-N-CH₂-CHO [(AO)ₘ₁R⁴]-CH₂-OR³ (1)
où
R¹ est un groupe polyhydroxyhydrocarbyle alcoxylé ; ou
R² est indépendamment un groupe tel que défini pour R¹ ; un groupe hydrocarbyle, en particulier alkyle ; alcoxyalkyle ; hydroxyalkyle alcoxylé éventuellement à coiffage en bout ; ou
R² est un groupe de formule : CH₂-CHO[(AO)ₘ₁R⁴]-CH₂-OR³ où AO, m1, R⁴ et R³ sont chacun indépendamment tels que définis ci-dessous;
R³ est un groupe hydrocarbyle en C₆ à C₃₀, en particulier en C₈ à C₃₀, plus particulièrement en C₁₀ à C₂₀, spécialement un groupe alkyle, alcényle, alcaryle, aryle ou aralkyle ;
chaque AO est indépendamment un groupe alkylèneoxy, en particulier un groupe alkylèneoxy en C₂ à C₄, spécialement un groupe alkylèneoxy en C₂ ou C₃, ou un mélange de groupes alkylèneoxy en C₂ et C₃ ;
m1 est compris entre 0 et 50, mais il est habituellement d'au moins 0,1, et de manière souhaitable, il est compris entre 0,5 et 20 ; et
R⁴ est un atome d'hydrogène ou un groupe alkyle ;
de sorte que le nombre total moyen de groupes alkylèneoxy dans la molécule est d'au moins 3.

2. Composé alcoxylé de la formule (II)
R^{1a}R^{2a}-N-CH₂-CHOH-CH₂-OR³ (II)
ou
R^{1a} est un groupe polyhydroxyhydrocarbyle ;
R^{2a} est indépendamment un groupe tel que défini pour R^{1a}, où est un groupe hydrocarbyle, en particulier alkyle ou alcoxyalkyle ou hydroxyalkyle, ou est un groupe de formule : -CH₂-CHOH-CH₂-OR³ où R³ est un groupe hydrocarbyle en C₆ à C₃₀,
dans lequel le nombre total moyen de groupes alkylèneoxy dans la molécule est d'au moins 3.

3. Composé selon la revendication 1, dans lequel R¹ est un groupe de formule (III) :
-R⁵[-O(AO)ₘ₃-R⁶]ₙ (III)
où
R⁵ est le résidu d'un groupe hydrocarbyle ;
R⁶ est un atome d'hydrogène ou un groupe alkyle ;
AO est un groupe alkylèneoxy ;
m3 est une valeur moyenne comprise entre 1 et 20 ; et
n est compris entre 3 et 10.

4. Composé selon la revendication 3, dans lequel R⁵ est le résidu (théoriquement obtenu par retrait des groupes hydroxyle du groupe parent) d'un groupe polyhydroxyalkyle ayant une chaîne en C₄ à C₇ linéaire.

5. Composé selon la revendication 3 ou la revendication 4, dans lequel n est compris entre 3 et 6.

6. Composé selon l'une quelconque des revendications 1 ou 2 à 4, dans lequel R² est un groupe alkyle et R³ est un groupe alkyle, alcényle, alcaryle, aryle ou aralkyle en C₁₀ à C₃₀·

7. Composé selon l'une quelconque des revendications 1 ou 2 à 5, dans lequel le ou les groupes alkylèneoxy AO sont des groupes éthylèneoxy, propylèneoxy ou des mélanges de groupes éthylèneoxy et propylèneoxy.

8. Composé selon la revendication 6, dans lequel le nombre total de groupes alkylèneoxy dans le composé de formule (I) est compris entre 5 et 30.

9. Composition agrochimique comportant en tant qu'adjuvant un composé de formule (I) selon l'une quelconque des revendications 1 à 8.

10. Composition selon la revendication 9, dans laquelle le composé actif sur le plan agrochimique est l'un ou plusieurs parmi des régulateurs de la croissance végétale, des herbicides et/ou des pesticides, par exemple des insecticides, des fongicides, des acaricides, des nématocides, des miticides, des rodenticides, des bactéricides, des molluscicides et/ou des répulsifs contre les oiseaux.

11. Composition selon la revendication 9, dans laquelle le composé actif d'un point de vue agrochimique est ou comporte au moins un herbicide soluble dans l'eau.

12. Composition selon la revendication 10, dans laquelle l'herbicide soluble dans l'eau est ou comporte au moins une phosphonométhylglycine ; au moins un acide aminé à groupe phosphinyle ; et/ou au moins un composé de bipyridinium.

13. Composition selon l'une quelconque des revendications 9 à 12, qui comporte de plus au moins un autre agent tensioactif.

14. Composition selon la revendication 13, qui comporte de plus au moins un agent tensioactif alkylpolysaccharidique.

15. Procédé de traitement d'une végétation par application d'une composition selon l'une quelconque des revendications 9 à 14 à des plantes et/ou un sol.

16. Procédé de destruction ou d'inhibition d'une végétation par application d'une formulation selon l'une quelconque des revendications 9 à 14, qui comporte un ou plusieurs parmi des régulateurs de la croissance et/ou des herbicides et au moins un composé de formule générale (I) tel que défini dans l'une quelconque des revendications 1 à 6 en tant qu'adjuvant.

17. Procédé de destruction d'organismes nuisibles aux végétaux par application d'une formulation selon l'une quelconque des revendications 9 à 14, qui comporte un ou plusieurs parmi des pesticides, des fongicides ou des acaricides, et au moins un composé de formule générale (I) tel que défini dans l'une quelconque des revendications 1 à 6 en tant qu'adjuvant.
